# EUROPEAN PATENT SPECIFICATION

(11) **EP 3 957 245 B1**
(45) Date of publication and mention of the grant of the patent: **01.01.2025**
(21) Application number: 21191449.4
(22) Date of filing: 16.08.2021
(51) Int. Cl.: A61B 5/287, A61B 5/339, A61B 5/367, A61B 5/00, A61B 18/14, A61B 17/00, A61B 18/00, A61B 34/20, A61B 90/00

(54) **REAL-TIME ASSESSMENT OF REJECTION FILTERS DURING CARDIAC MAPPING**
ECHTZEITBEURTEILUNG VON SPERRFILTERN WÄHREND DER HERZABBILDUNG
ÉVALUATION EN TEMPS RÉEL DE FILTRES DE REJET PENDANT LA CARTOGRAPHIE CARDIAQUE

(30) Priority: 17.08.2020 US 202016995036
(43) Date of publication of application: 23.02.2022
(73) Proprietor: Biosense Webster (Israel) Ltd., Yokneam 2066717 (IL)
(72) Inventor: Palti, Yair, Yokneam (IL); Govari, Assaf, Yokneam (IL); Gliner, Vadim, Yokneam (IL); Avni, Uri, Yokneam (IL); Salevich, Alexander, Yokneam (IL)
(74) Representative: Carpmaels & Ransford LLP

(56) References cited:
- EP-A1- 2 462 867
- US-A1- 2011 137 153
- US-A1- 2015 057 507

## Description

### FIELD OF THE INVENTION

The present invention relates generally to cardiac electrophysiological (EP) mapping, and particularly to graphical user interfaces (GUI) for cardiac EP mapping.

### BACKGROUND OF THE INVENTION

Various techniques for visually analyzing EP data were reported in the patent literature. For example, U.S. Patent No. 8,478,393 describes a method for visualization of electrophysiology data representing electrical activity on a surface of an organ over a time period. An interval within the time period is selected in response to a user selection. Responsive to the user selection of the interval, a visual representation of physiological information for the user selected interval is generated by applying at least one method to the data. The visual representation is spatially represented on a graphical representation of a predetermined region of the surface of the organ.

US 2015/057507 A1 discloses an electrophysiology map that can be generated from a plurality of electrophysiology data points added automatically in response to defined inclusion criteria. Inclusion criteria can generally be grouped into two categories: location-based (e.g., velocity, distance moved, dwell time, and proximity) and rhythm-based (e.g., cycle length and EKG matching). As each electrophysiology data point is collected, it can be tested against one or more defined inclusion criteria, and added to the electrophysiology map when it satisfies all such criteria. Inclusion criteria can also be employed to generate the geometric model underlying the electrophysiology map.

### SUMMARY OF THE INVENTION

The invention is defined by the appended claims. 1 An embodiment of the present invention that is described hereinafter provides a system including a display and a processor. The processor is configured to (a) receive multiple electrophysiological (EP) signals acquired by multiple electrodes of a multi-electrode catheter that are in contact with tissue of a cardiac chamber, (b) reject one or more of the EP signals using a set of rejection criteria, and further process the EP signals that are not rejected, and (c) visualize to a user, on the display, (i) a current setting of the rejection criteria, and (ii) a rejection

effectiveness of each of the rejection criteria at the current setting.

In some embodiments, the system further includes an input device, and the processor is configured to receive, via the input device, user input that reconfigures the setting of one or more of the rejection criteria in response to the visualized rejection effectiveness.

In some embodiments, the processor is configured to visualize the rejection effectiveness by plotting at least some of the EP signals, and mark a rejected EP signal with a mark indicative of a rejection criterion used for rejecting the EP signal.

In other embodiments, the processor is configured to visualize the rejection effectiveness by graphically illustrating the multiple electrodes of the multi-electrode catheter, and, for a rejected EP signal, mark an electrode used to acquire the rejected EP signal with a mark indicative of a rejection criterion used for rejecting the EP signal.

In an embodiment, the processor is further configured to graphically illustrate an orientation of the multiple electrodes relative to an anatomy of the cardiac chamber.

In some embodiments, the processor is configured to visualize the rejection effectiveness in real-time. In other embodiments, the processor is configured to visualize a given rejection criteria and the rejection effectiveness of the given rejection criteria, using a same graphical feature.

In an embodiment, the graphical feature includes one or more of a color and a pattern.

There is additionally provided, in accordance with another embodiment, not claimed as such, but nevertheless useful for understanding the present invention, a method

including receiving multiple electrophysiological (EP) signals acquired by multiple electrodes of a multi-electrode catheter that are in contact with tissue of a cardiac chamber. One or more of the EP signals are rejected using a set of rejection criteria, and further processing the EP signals that are not rejected. Visualized to a user, on a display, are (i) a current setting of the rejection criteria, and (ii) a rejection effectiveness of each of the rejection criteria at the current setting.

There is further provided, in accordance with another embodiment of the present invention, a computer software product, the product including a tangible non-transitory computer-readable medium in which program instructions are stored, which instructions, when read by a processor in a cardiac diagnostic and therapeutic system, cause the processor to (a) receive multiple electrophysiological (EP) signals acquired by multiple electrodes of a multi-electrode catheter that are in contact with tissue of a cardiac chamber, (b) reject one or more of the EP signals using a set of rejection criteria, and further process the EP signals that are not rejected, and (c) visualize to a user, on a display, (i) a current setting of the rejection criteria, and (ii) a rejection effectiveness of each of the rejection criteria at the current setting.

### BRIEF DESCRIPTION OF THE DRAWINGS

The present invention will be more fully understood from the following detailed description of the embodiments thereof, taken together with the drawings in which:
Fig. 1 is a schematic, pictorial illustration of an electrophysiological (EP) mapping system comprising different possible multi-electrode catheters, in accordance with exemplary embodiments of the present invention;
Fig. 2 is a schematic illustration of a graphical user interface (GUI) of the electrophysiological (EP) mapping system of Fig. 1, in accordance with an exemplary embodiment of the present invention; and
Fig. 3 is a flow chart that schematically illustrates a method for distinguishing among filters in real time, using the graphical user interface (GUI) of Fig. 2, in accordance with an exemplary embodiment, not claimed as such, but nevertheless useful for understanding the present invention.

### DETAILED DESCRIPTION OF EMBODIMENTS

### OVERVIEW

Probe-based (e.g., catheter-based) cardiac diagnostic and therapeutic systems may measure multiple intra-cardiac electrophysiological (EP) signals, such as electrograms (EGM), during an invasive procedure. Such systems acquire multiple intra-cardiac signals using multiple electrodes (also referred to hereinafter as "distal electrodes") that are fitted at the distal end of the probe. The analysis of acquired EP signals, in most cases, is done in continuous mode (automatically) in order to be able to process vast amounts of EP information.

Automation of the analysis often involves applying rejection criteria automatically (e.g., the use of automatic "filtering" to avoid using "wrong" channels (e.g., irrelevant, too noisy) at any given time. The physician performing the procedure therefore needs to set "good" filtering criteria in order to analyze only "good" (e.g., relevant, stable) channels. The measured and filtered signals may be analyzed in real time to provide the physician with visual cardiac information, such as 3-D mapping of the source of pathological electrical patterns within the heart of the patient. Three-dimensional mapping may be used to support corrective medical procedures, such as *in situ* ablation (e.g., using the same catheter). Additionally, or alternatively, the measured and filtered signals may be subsequently analyzed, e.g., offline.

In more detail, to reduce the time required to EP map a heart chamber, e.g., to acquire EGM signals of the chamber, a catheter carrying a large number of electrodes (e.g., 256), such as a basket catheter, may be used. The acquired signals should typically be subjected to rejection criteria to remove signals that may be incorrect (e.g., unstable) or irrelevant (e.g., acquired from the blood pool instead of the chamber surface). For large numbers of electrodes, applying rejection criteria may be done automatically by setting levels of individual filter scales shown on a display. However, it is difficult for a physician to tell whether the filter setting levels are reasonable, or whether too many signals are being rejected.

Embodiments of the present invention that are described hereinafter provide visual real-time assessment of the effectiveness of rejection criteria. In some embodiments, a graphical user interface (GUI) provides a preferences window that shows different rejection criteria that can be adjusted (e.g., filters with scales that can be dialed) by the physician. Another GUI window shows the EP signals acquired by respective electrodes of a multi-electrode catheter. Whenever a filter rejects an acquired EP signal, the signal is marked by distinctive graphics (e.g., a color) of, for example, the scale of the filter by which it was rejected.

In another embodiment, a third window of the GUI shows the electrodes in relation to the anatomical surface being mapped. The electrodes are patterned (e.g., colored) by the disclosed GUI with the same distinctive graphics of the filter and the respective rejected EP signals. In other words, when a filter rejects an acquired EP signal, the electrode used for acquiring the signal is marked by the distinctive graphics of the filter by which the signal was rejected.

Further alternatively, the rejection effectiveness of the rejection criteria can be visualized to the physician in any other suitable way.

All or part of the above visualization methods allow the physician to investigate and change (e.g., dial a level of) a filter scale to easily optimize filtration settings. Therefore, the disclosed technique allows the visualization of how each filter criterion influences the signals to be used for mapping, and those which will not be used, all in real-time.

In some embodiments, the rejection criteria (e.g., filter scales limits) can be modified by dialing a threshold, or dialing a range (e.g., by dialing low and high limits) to generate criteria for signal rejection. A processor of the mapping system compares real-time values of each data point (e.g., an annotated signal) to the dialed criteria and acts accordingly to reject or accept the data point.

For example, in one embodiment, any captured data point having a bipolar voltage amplitude value lower than a dialed signal amplitude threshold (e.g., below 0.1 mV) is filtered out (i.e., rejected), while the acquiring electrode-pair, channel, and the waveform are given the same color of the respective filter. In a second embodiment, any captured data point having a cycle length outside a dialed range (e.g., between 600 mS and 900 mS) is filtered out and the electrode, channel, and waveform are given the same color of the filter.

In a third embodiment, any captured data point using a dialed criterion is deemed an outlier (e.g., not within a dialed range of a prespecified parameter from data points of surrounding electrodes) and is filtered out, and the electrode, channel, and the waveform are given the same color of the respective filter. In a fourth embodiment, the current position of any captured data point whose location is more than a dialed value (e.g., 2 mm) from the electrode's previous location is filtered out, and the capturing electrode, channel, and waveform are given the same color of the respective filter.

Other rejection (e.g., filtration) criteria may also be employed to reject data points in real time using a similar visualization for rejection. Such further filtration criteria include unstable local activation time (LAT) value, weak physical contact between electrode and tissue, and low measured impedance (e.g., that of blood instead of that of tissue). Following rejection, the respective electrode and signal are given the same color as the rejecting filter.

In an embodiment, the disclosed GUI visualizes to a user, on the display, (i) a current setting of the rejection criteria, and (ii) a rejection effectiveness of each of the rejection criteria at the current setting. The processor may visualize the rejection effectiveness by plotting at least some of the EP signals, and mark a rejected EP signal with a mark indicative of a rejection criterion used for rejecting the EP signal. Similarly, the processor may visualize the rejection effectiveness by graphically illustrating the multiple electrodes of the multi-electrode catheter, and, for a rejected EP signal, mark an electrode used to acquire the rejected EP signal with a mark indicative of a rejection criterion used for rejecting the EP signal.

For example, an icon in the GUI summarizes the actions of the different filters, for example, using a "funnel" icon through which filtration statistics are "poured" (e.g., into a "measuring tube" icon). The information on the effectiveness in the funnel icon is coded into two parts, one of which shows the user how many channels are filtered by which filters (represented by colored portions of the funnel which are graphically coded to the respective filter color), and the other showing the dominant filter by using the enclosed coded text of the filtered-out channel.

In another embodiment, a color bar on the GUI shows the percentage of data points of each filter type that pass a given filtration, using the same graphics (e.g., color) of the filter.

In some embodiments, the physician uses an input device, such as a computer mouse, to reconfigure the setting of one or more of the rejection criteria in response to the visualized rejection effectiveness, for example by changing in a filter scale a range within signal is accepted and be further processed.

By providing a GUI comprising a set of filter scales that can be adjusted (e.g., dialed) in real time, a physician can optimize, in situ (during EP mapping), the quality of the analysis, thereby improving the diagnostic quality of the invasive procedure.

### SYSTEM DESCRIPTION

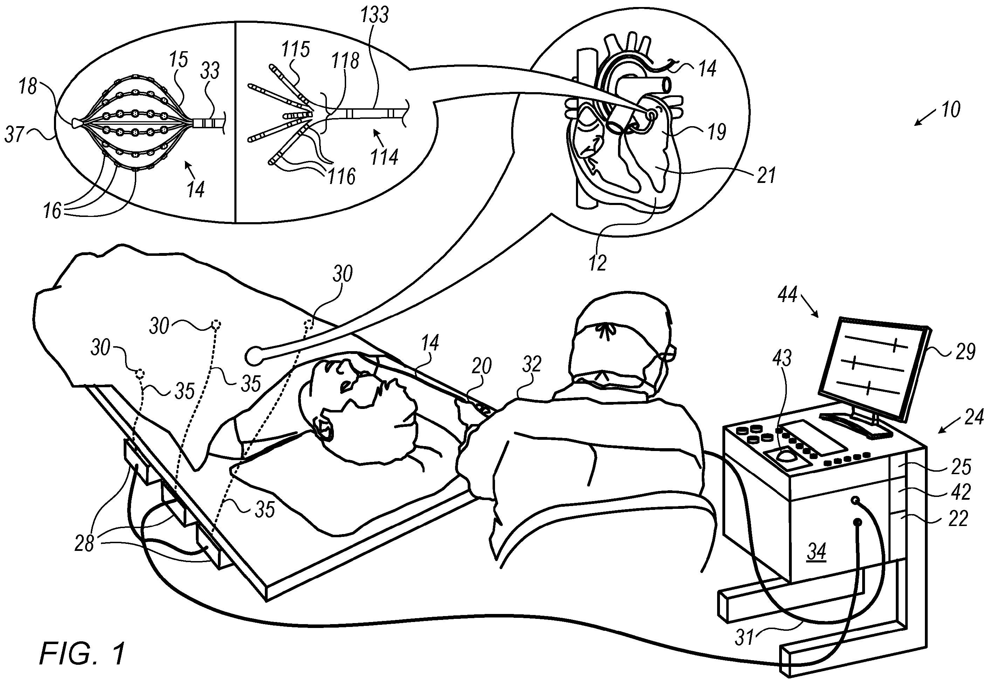

Fig. 1 is a schematic, pictorial illustration of an electrophysiological (EP) mapping system 10 comprising different possible multi-electrode catheters, in accordance with exemplary embodiments of the present invention. System 10 may be configured to analyze substantially any physiological parameter or combinations of such parameters. In the description herein, by way of example, the analyzed signals are assumed to be intra-cardiac electrogram potential-time relationships. In order to fully characterize such relationships, the signals at various locations must be referenced in time to each other, such as is done during LAT map generation. This time referencing is accomplished by taking measurements relative to a reference time (e.g., an instance in time), such as the beginning of each QRS complex of an ECG reference signal (i.e., the beginning of every heartbeat). The method for generating an LAT map is described in U.S. Patent No. 9,050,011, cited above.

As noted above, system 10 comprises a multi-electrode catheter, which can be, among numerous possible options, a basket catheter 14 or a multi-arm catheter 114 (e.g., a PentaRay^{™} catheter), both of which are shown in inset 37. The description hereinafter collectively calls the above catheter options "catheter 14/114," which means the embodiments described hereinafter hold for either of these multi-electrode catheter types.

Multi-electrode catheter 14/114 is inserted by a physician 32 through the patient's vascular system into a chamber or vascular structure of a heart 12. Physician 32 brings the catheter's distal tip 18/118 into contact with (e.g., presses the tip distally against) wall tissue 19 of a cardiac chamber 21, at an EP mapping target tissue site. The catheter typically comprises a handle 20 which has suitable controls to enable physician 32 to steer, position, and orient the distal end of the catheter as desired for EP mapping.

Multi-electrode catheter 14/114 is coupled to a console 24, which enables physician 32 to observe and regulate catheter functions. To aid physician 32, the distal portion of the catheter may contain various sensors, such as contact force sensors (not shown) and a magnetic sensor 33/133 that provides position, direction, and orientation signals to a processor 22, located in the console 24. Processor 22 may fulfill several processing functions as described below. In particular, electrical signals can be conveyed to and from heart 12 from electrodes 16/116 located at or near the distal tip 18/118 of catheter 14/114 via cable 31 to console 24. Pacing signals and other control signals may be conveyed from console 24 through cable 31 and electrodes 16/116 to heart 12.

Console 24 includes a monitor 29 driven by processor 22. Signal processing circuits in an electrical interface 34 typically receive, amplify, analog-filter, and digitize signals from catheter 14/114 to produce multiple digital signals, including signals generated by the above-noted plurality of sensing electrodes 16/116. The digitized signals are received and used by console 24 and the positioning system to compute the position and orientation of catheter 14/114 and to analyze EP signals from electrodes 16/116, as described in further detail below.

During the EP mapping procedure, a tracking system is used to track the intra-cardiac locations of distal electrodes 16/116, so that each of the acquired EP signals may be associated with a known intra-cardiac location. An example of such a tracking system is Active Current Location (ACL), which is described in U.S. Patent No. 8,456,182, which is assigned to the assignee of the present patent application. In the ACL system, a processor estimates the respective locations of distal electrodes 16/116 based on impedances measured between each of distal electrodes 16/116 and a plurality of surface electrodes 30 that are coupled to the skin of the patient and wired (35) to console 24. The processor may then associate any electrophysiological signal received from distal electrodes 16/116 with the location at which the signal was acquired.

In alternative embodiments, position measurements can also be done by applying a voltage gradient between pairs of surface electrodes 30 and measuring, with distal electrodes 16/116, the resulting potential gradients.

In some embodiments, system 10 comprises, in addition to, or instead of, the ACL tracking subsystem, a magnetic position tracking subsystem that determines the position and orientation of magnetic sensor 33, at a distal end of catheter 14/114, by generating magnetic fields in a predefined working volume, and sensing these fields at the catheter using field generating coils 28. As electrodes 16/116 have known locations on arms 15/115, and known relationships to one to the other, once catheter 14/114 is tracked magnetically in the heart, the location of each of electrodes 16/116 in the heart becomes known. A suitable magnetic position tracking subsystem is described in U.S. Patent Nos. 7,756,576 and 7,536,218, which are assigned to the assignee of the present patent application.

Based on the EP signals from electrodes 16/116 having tracked locations, electrical activation maps may be prepared, according to the methods disclosed in U.S. Patent Nos. 6,226,542, and 6,301,496, and 6,892,091, which are assigned to the assignee of the present patent application.

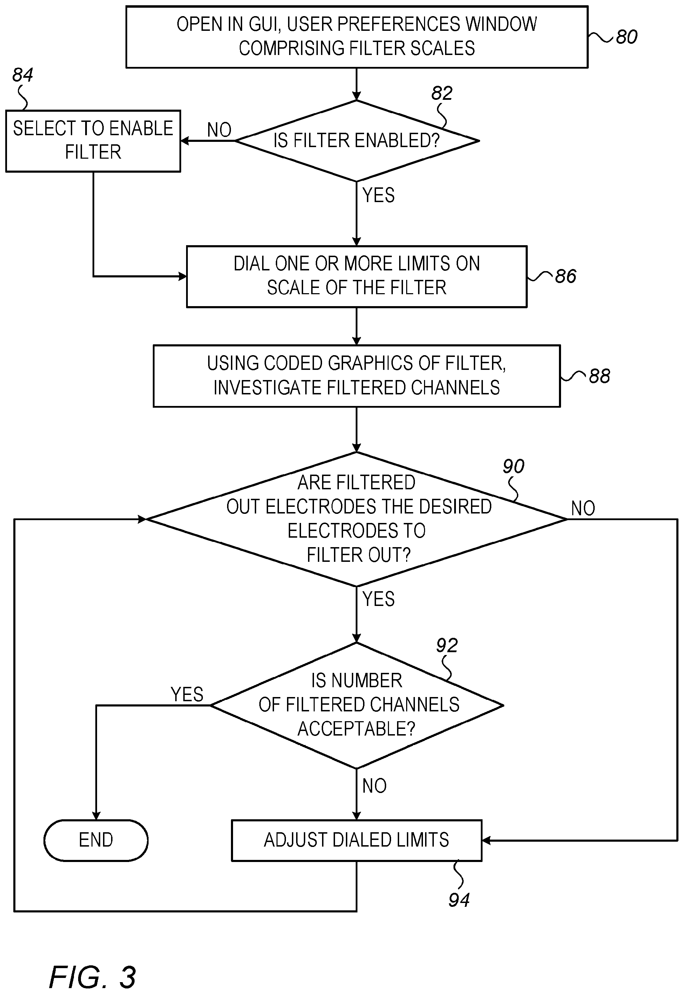

Processor 22 uses software stored in a memory 25 to operate system 10. The software may be downloaded to processor 22 in electronic form, over a network, for example, or it may, alternatively or additionally, be provided and/or stored on non-transitory tangible media, such as magnetic, optical, or electronic memory. In particular, processor 22 runs a dedicated algorithm as disclosed herein, including in Fig. 3, that enables processor 22 to perform the disclosed steps, as further described below.

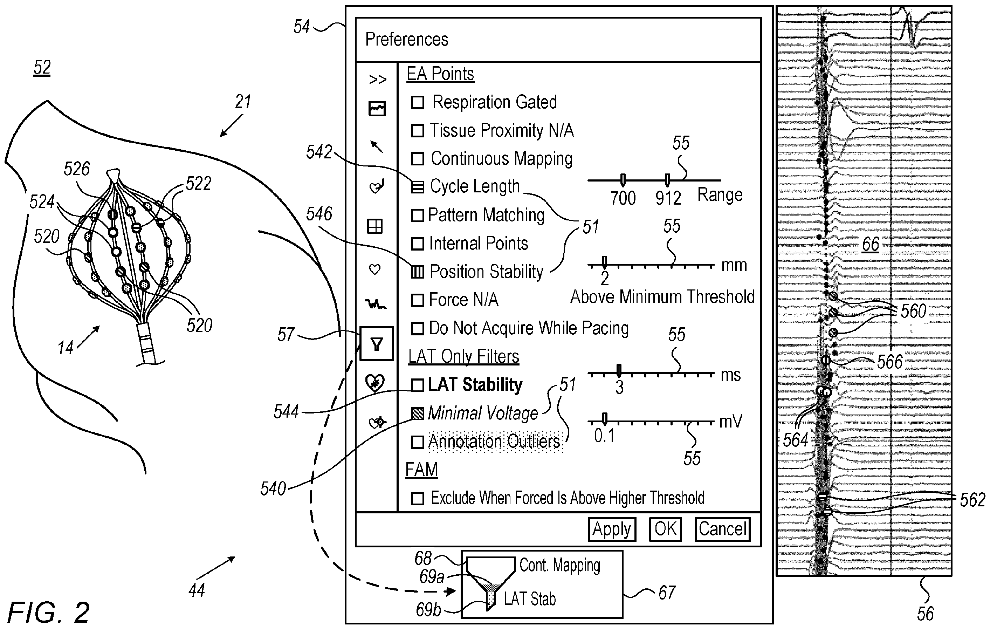

Processor 22 comprises a signal-processing unit 42 that is configured to digitally filter the multi-channel signals and extract respective annotation parameters from the signals. Digital filters of unit 42 can be dialed, using, for example, an input device that is one of a computer mouse 43, a keyboard, and a touch display, and using graphical user interface (GUI) 44, to accept or reject electrogram signals based the dialed level of the digital filter, as seen in Fig. 2. Examples of dialed filters levels include cycle-length, LAT stability, and minimal signal voltage, to name a few.

The example illustration shown in Fig. 1 is chosen purely for the sake of conceptual clarity. Other types of EP sensing geometries, such as of a balloon catheter comprising electrode segments, described in U.S. Patent Application No. 16/708285, titled, "Catheter with Plurality of Sensing Electrodes Used as Ablation Electrodes," filed December 9, 2019, may also be employed.

Further types of catheters, such as the Lasso^{®} Catheter (produced by Biosense-Webster), may equivalently be employed. Other types of electrodes, such as those used for ablation, may be utilized in a similar way to electrodes 16/116 to acquire intra-cardiac electrophysiological signals.

System 10 typically comprises additional modules and elements that are not directly related to the disclosed techniques, and thus are intentionally omitted from Fig. 1 and from the corresponding description. The elements of system 10 and the methods described herein may be further applied, for example, to control an ablation of tissue of heart 12.

### DISTINGUISHING BETWEEN FILTERS DURING CARDIAC MAPPING IN REAL TIME

Fig. 2 is a schematic illustration of a graphical user interface (GUI) 44 of the electrophysiological (EP) mapping system 10 of Fig. 1, in accordance with an embodiment of the present invention. In the shown embodiment, GUI 44 comprises filter scales 55 that can be adjusted by a user during EP mapping.

In the shown embodiment, GUI 44 displays three windows (52, 54 and 56). Window 52 shows basket catheter 14 inside a cardiac chamber 21 where the catheter is being used for EP mapping of chamber 21 wall tissue to, for example, detect arrhythmogenic tissue. The catheter is shown with the orientation of its multiple electrode relative to an anatomy of a cardiac chamber. As seen, some of the electrodes of the catheter are coded with distinctive graphics, the same codes as those used by filters in window 54.

Window 54 shows a user preferences menu comprising different filter scales 55 with the respective filter names appearing to the left. Respective checkboxes enable a user to activate or deactivate each filter.

Each enabled filter has a color-coded name (51) and distinctive graphics (e.g., codes 540, 542, 544, and 546) with which it is associated. Electrodes with filtered-out signals are coded with the distinctive graphics of the filter (e.g., codes 520, 522, 524, and 526), as can be seen, by way of example, in the embodiment below:
Electrode coded by graphics 520 has its acquired signal rejected by a minimal voltage filter, coded 540.
Electrode coded by graphics 522 has its acquired signal rejected by a cycle-length-range filter, coded 542.
Electrode coded by graphics 524 has its acquired signal rejected by unstable LAT value filter, coded 544.
Electrode coded by graphics 526 has its acquired signal rejected by unstable electrode-position filter, coded 546.

Window 56 displays acquired electrograms (66) and filtered-out electrograms that are given the same graphical code as the filters by which they were rejected (e.g., by codes 560, 562, 564, and 566). As seen in window 56, the electrograms are annotated by a graphically coded point at a location on the electrogram portion where the analysis was performed, typically at or near the QRST complex of a heart beat cycle captured by the electrogram.

In an embodiment, the user receives a summary of the actions of the filters at any given time, in the form of icon 67 that is selected by pressing icon 57 in the preferences menu. As seen, icon 67 has a funnel icon 68 via which filtration statistics are "poured" (into a measuring tube icon, not shown). The information in funnel icon 68 is coded into two parts, the first showing the user how many channels are filtered by which filter, indicated by the amount of a colored portions (two shown: 69a, 69b) of the funnel that is graphically coded the same as the respective filter), and the second showing the dominant filter, indicated by the enclosed coded text, e.g., "LAT stability" filtering a dominant portion 69b of the filtered-out channels.

Fig.2 is an example brought solely for the sake of describing an embodiment. An actual GUI is typically far more elaborate, including, for example, numerous icons and graphics that are omitted herein for simplicity of presentation. As another example, GUI 44 typically includes various types of menus and overlaid information that are omitted for simplicity of presentation.

Fig. 3 is a flow chart that schematically illustrates a method for distinguishing among filters in real time, using graphical user interface (GUI) 44 of Fig. 2, in accordance with an embodiment, not claimed as such, but nevertheless useful for understanding the present invention. The flow chart describes a workflow, including dialing a scale of a given filter, though it should be understood that a
user may adjust several filters while investigating a relative contribution of each filter to the total count of channels being filtered out.

The algorithm, according to the presented embodiment, carries out a process that begins after physician 32 brings basket catheter 14 into a target tissue location in cardiac chamber 21, where, at GUI operation step 80, physician 32 opens preferences window 54 on display 29.

Next, physician 32 checks if a required filter is enabled, at a checking filter menu step 82. If the filter is not enabled, physician 32 enables the filter by selecting it (e.g., by clicking a checkbox), at a filter enablement step 84. Depending on the type of the filter (e.g., filtering based on threshold or acceptable range), physician 32 adjusts (e.g., dials) one or more limits, at a filter scale dialing step 86.

Next, physician 32 investigates the filtering results, at a channel filtration checking step 88. The physician may, for example, investigate the tissue location and number of channels affected by the filter operation.

For example, in electrode filtration out checking step 90, the physician examines, in window 52 of GUI 44, the identity (e.g., position) of the electrodes filtered out by the given filter. These electrodes are easily identified as they are coded by a same graphical code (e.g., color) of the filter, as described in Fig. 2.

If physician 32 finds, by looking are window 52, that at least part of the filtered-out electrodes are in fact located in place and presumably are acquiring valid signals, the physician adjusts the one or more limits on the filter scale to make the filter less aggressive, at a scale adjustment step 94.

If, however, the physician identifies on window 52 that filtered-out electrodes are at a location that have correctly been filtered out (e.g., they are immersed in blood), the process continues to a step 92. At channel filtering out checking step 92, the physician checks an absolute number or fraction of filtered-out electrodes by the filter.

If physician 32 finds, by looking at icon 67, or by looking in window 56, that at least part of the filtered-out channels is too large, and/or that the waveforms are valid, the physician adjusts the one or more limits on the filter scale to make the filter less aggressive, at a scale adjustment step 94.

The process then returns to step 90 for the physician to verify that the adjusted filter scale did not adversely affect the filtering out of electrodes.

The process ends when the physician finds the identity of the filtered-out electrodes and the number of channels (e.g., number of electrodes) to be sufficiently optimal.

The example flow chart of Fig. 3 is chosen purely for the sake of conceptual clarity. In alternative embodiments, for example, the physician adjusts a scale of at least one additional filter and investigates the accumulative filtering out effect of the two filters.

It will be appreciated that the embodiments described above are cited by way of example, and that the present disclosure a is not limited to what has been particularly shown and described hereinabove. Rather, the scope of the present disclosure includes both combinations and subcombinations of the various features described hereinabove.

The scope of the present invention is nevertheless defined by the appended claims.

## Claims

1. A cardiac diagnostic and therapeutic system (10), comprising:
a display (29); and
a processor (22), configured to:
receive multiple electrophysiological (EP) signals (66) acquired by multiple electrodes (16, 116) of a multi-electrode catheter (14, 114) that are in contact with tissue (19) of a cardiac chamber (21);
reject one or more of the EP signals using a set of rejection criteria, and further process the EP signals that are not rejected; and
visualize to a user (32), on the display, (i) a current setting (54) of the rejection criteria, and (ii) a rejection effectiveness of each of the rejection criteria at the current setting.

2. The system according to claim 1, and comprising an input device (43), wherein the processor is configured to receive, via the input device, user input (94) that reconfigures the setting of one or more of the rejection criteria in response to the visualized rejection effectiveness.

3. The system according to claim 1, wherein the processor is configured to visualize the rejection effectiveness by plotting (56) at least some of the EP signals, and mark a rejected EP signal with a mark (560, 562, 564, 566) indicative of a rejection criterion (540, 542, 544, 546) used for rejecting the EP signal.

4. The system according to claim 1, wherein the processor is configured to visualize the rejection effectiveness by graphically illustrating (52) the multiple electrodes of the multi-electrode catheter, and, for a rejected EP signal, mark an electrode used to acquire the rejected EP signal with a mark (520, 522, 524, 526) indicative of a rejection criterion (540, 542, 544, 546) used for rejecting the EP signal.

5. The system according to claim 4, wherein the processor is further configured to graphically illustrate an orientation of the multiple electrodes relative to an anatomy of the cardiac chamber.

6. The system according to claim 1, wherein the processor is configured to visualize the rejection effectiveness in real-time.

7. The system according to claim 1, wherein the processor is configured to visualize a given rejection criteria and the rejection effectiveness of the given rejection criteria, using a same graphical feature.

8. The system according to claim 6, wherein the graphical feature comprises one or more of a color and a pattern.

9. A computer software product, the product comprising a tangible non-transitory computer-readable medium in which program instructions are stored, which instructions, when read by a processor (22) in a cardiac diagnostic and therapeutic system (10), cause the processor to:
receive multiple electrophysiological (EP) signals (66) acquired by multiple electrodes (16, 116) of a multi-electrode catheter (14, 114) that are in contact with tissue (19) of a cardiac chamber (21);
reject one or more of the EP signals using a set of rejection criteria, and further process the EP signals that are not rejected; and
visualize to a user (32), on a display, (i) a current setting (54) of the rejection criteria, and (ii) a rejection effectiveness of each of the rejection criteria at the current setting.

## Patentansprüche

1. Herzdiagnose- und -therapiesystem (10), umfassend:
eine Anzeige (29); und
einen Prozessor (22), der konfiguriert ist zum:
Empfangen mehrerer elektrophysiologischer (EP) Signale (66), die durch mehrere Elektroden (16, 116) eines Mehrelektrodenkatheters (14, 114) erfasst werden, die mit Gewebe (19) einer Herzkammer (21) in Kontakt stehen;
Ablehnen eines oder mehrerer EP-Signale unter Verwendung eines Satzes von Ablehnungskriterien und ferner Verarbeiten der EP-Signale, die nicht abgelehnt werden; und
Visualisieren einem Benutzer (32), auf der Anzeige (i), einer aktuellen Einstellung (54) der Ablehnungskriterien und (ii) einer Ablehnungswirksamkeit jedes der Ablehnungskriterien bei der aktuellen Einstellung.

2. System nach Anspruch 1, und umfassend eine Eingabevorrichtung (43), wobei der Prozessor konfiguriert ist, um, über die Eingabevorrichtung, eine Benutzereingabe (94) zu empfangen, die die Einstellung eines oder mehrerer Ablehnungskriterien als Reaktion auf die visualisierte Ablehnungswirksamkeit neu konfiguriert.

3. System nach Anspruch 1, wobei der Prozessor konfiguriert ist, um die Ablehnungswirksamkeit durch Plotten (56) von mindestens einigen der EP-Signale zu visualisieren und ein abgelehntes EP-Signal mit einer Markierung (560, 562, 564, 566) zu markieren, die ein Ablehnungskriterium (540, 542, 544, 546) angibt, das zum Ablehnen des EP-Signals verwendet wird.

4. System nach Anspruch 1, wobei der Prozessor konfiguriert ist, um die Ablehnungswirksamkeit durch grafisches Darstellen (52) der mehreren Elektroden des Mehrelektrodenkatheters zu visualisieren, und, für ein abgelehntes EP-Signal eine Elektrode zu markieren, die verwendet wird, um das abgelehnte EP-Signal mit einer Markierung (520, 522, 524, 526) zu erfassen, die ein Ablehnungskriterium (540, 542, 544, 546) angibt, das zum Ablehnen des EP-Signals verwendet wird.

5. System nach Anspruch 4, wobei der Prozessor ferner konfiguriert ist, um eine Ausrichtung der mehreren Elektroden relativ zu einer Anatomie der Herzkammer grafisch darzustellen.

6. System nach Anspruch 1, wobei der Prozessor konfiguriert ist, um die Ablehnungswirksamkeit in Echtzeit zu visualisieren.

7. System nach Anspruch 1, wobei der Prozessor konfiguriert ist, um ein gegebenes Ablehnungskriterium und die Ablehnungswirksamkeit des gegebenen Ablehnungskriteriums unter Verwendung eines gleichen grafischen Merkmals zu visualisieren.

8. System nach Anspruch 6, wobei das grafische Merkmal eines oder mehrere von einer Farbe und einem Muster umfasst.

9. Computersoftwareprodukt, das Produkt umfassend ein greifbares, nicht flüchtiges, computerlesbares Medium, auf dem Programmanweisungen gespeichert sind, wobei die Anweisungen, wenn sie durch einen Prozessor (22) in einem Herzdiagnose- und -therapiesystem (10) gelesen werden, den Prozessor veranlassen zum:
Empfangen mehrerer elektrophysiologischer (EP) Signale (66), die durch mehrere Elektroden (16, 116) eines Mehrelektrodenkatheters (14, 114) erfasst werden, die in Kontakt mit Gewebe (19) einer Herzkammer (21) stehen;
Ablehnen eines oder mehrerer EP-Signale unter Verwendung eines Satzes von Ablehnungskriterien und ferner Verarbeiten der EP-Signale, die nicht abgelehnt werden; und
Visualisieren einem Benutzer (32), auf einer Anzeige (i), einer aktuellen Einstellung (54) der Ablehnungskriterien und (ii) einer Ablehnungswirksamkeit jedes der Ablehnungskriterien bei der aktuellen Einstellung.

## Revendications

1. Système thérapeutique et de diagnostic cardiaque (10), comprenant :
un affichage (29) ; et
un processeur (22), configuré pour :
recevoir de multiples signaux électrophysiologiques (EP) (66) acquis par de multiples électrodes (16, 116) d'un cathéter à électrodes multiples (14, 114) qui sont en contact avec des tissus (19) d'une cavité cardiaque (21) ;
rejeter un ou plusieurs des signaux EP en utilisant un ensemble de critères de rejet, et traiter en outre des signaux EP qui ne sont pas rejetés ; et
représenter pour un utilisateur (32), sur l'affichage, (i) un réglage actuel (54) des critères de rejet, et (ii) une efficacité de rejet de chacun des critères de rejet au réglage actuel.

2. Système selon la revendication 1, et comprenant un dispositif d'entrée (43), dans lequel le processeur est configuré pour recevoir, par l'intermédiaire du dispositif d'entrée, une entrée utilisateur (94) qui reconfigure le réglage d'un ou de plusieurs des critères de rejet en réponse à l'efficacité de rejet représentée.

3. Système selon la revendication 1, dans lequel le processeur est configuré pour représenter l'efficacité de rejet en traçant (56) au moins certains des signaux EP, et pour marquer un signal EP rejeté d'une marque (560, 562, 564, 566) indiquant un critère de rejet (540, 542, 544, 546) utilisé pour rejeter le signal EP.

4. Système selon la revendication 1, dans lequel le processeur est configuré pour représenter l'efficacité de rejet en illustrant graphiquement (52) les multiples électrodes du cathéter à électrodes multiples et, pour un signal EP rejeté, pour marquer une électrode utilisée pour acquérir le signal EP rejeté d'une marque (520, 522, 524, 526) indiquant un critère de rejet (540, 542, 544, 546) utilisé pour rejeter le signal EP.

5. Système selon la revendication 4, dans lequel le processeur est en outre configuré pour illustrer graphiquement une orientation des multiples électrodes par rapport à une anatomie de la cavité cardiaque.

6. Système selon la revendication 1, dans lequel le processeur est configuré pour représenter l'efficacité de rejet en temps réel.

7. Système selon la revendication 1, dans lequel le processeur est configuré pour représenter un critère de rejet donné et l'efficacité de rejet du critère de rejet donné en utilisant une même caractéristique graphique.

8. Système selon la revendication 6, dans lequel la caractéristique graphique comprend un ou plusieurs parmi un motif et une couleur.

9. Produit logiciel informatique, le produit comprenant un support tangible non transitoire lisible par ordinateur dans lequel sont stockées des instructions de programme, lesquelles instructions, lorsqu'elles sont lues par un processeur (22) dans un système thérapeutique et de diagnostic cardiaque (10), amènent le processeur à :
recevoir de multiples signaux électrophysiologiques (EP) (66) acquis par de multiples électrodes (16, 116) d'un cathéter à électrodes multiples (14, 114) qui sont en contact avec
des tissus (19) d'une cavité cardiaque (21) ;
rejeter un ou plusieurs des signaux EP en utilisant un ensemble de critères de rejet, et traiter en outre des signaux EP qui ne sont pas rejetés ; et
représenter pour un utilisateur (32), sur un affichage, (i) un réglage actuel (54) des critères de rejet, et (ii) une efficacité de rejet de chacun des critères de rejet au réglage actuel.
